# EUROPEAN PATENT SPECIFICATION

(11) **EP 0 497 759 B1**
(45) Date of publication and mention of the grant of the patent: **15.02.1995**
(21) Application number: 90901193.4
(22) Date of filing: 20.11.1989
(51) Int. Cl.: C07C 53/12, C07C 51/573, C07C 51/56

(54) **PROCESS FOR THE RECOVERY OF CATALYST VALUES**
VERFAHREN ZUR WIEDERGEWINNUNG VON KATALYSATORWERTEN
PROCEDE DE RECUPERATION DE QUANTITES CATALYTIQUES

(43) Date of publication of application: 12.08.1992
(73) Proprietor: EASTMAN CHEMICAL COMPANY, Kingsport, TN 37660 (US)
(72) Inventor: ODOM, Steve, Conley, Rochester, New York 14650 (US); Cook, Steven Leroy, Rochester, New York 14650 (US); Tennant, Brent Alan, Rochester, New York 14650 (US)
(74) Representative: Behrens, Dieter, Dr.-Ing.
(86) International application number: US8905193
(87) International publication number: WO9107372

(56) References cited:
- EP-A- 0 200 023
- US-A- 4 364 907

## Description

This invention pertains to an improved process for recovering rhodium catalyst values. More specifically, this invention pertains to a process for the recovery of rhodium from "tars" formed during the preparation of acetic anhydride by the rhodium-catalyzed carbonylation of a mixture of methyl iodide and methyl acetate.

The use of catalyst systems comprising rhodium and an iodine compound in the preparation of acetic anhydride by the carbonylation of methyl acetate has been reported extensively in the patent literature. See, for example, U.S. Patents 3,927,078, 4,046,807, 4,374,070 and 4,559,183 and European Patents 8396 and 87,870. These patents disclose that the reaction rate can be increased if the catalyst system includes a promoter such as certain amines, quaternary ammonium compounds, phosphines and inorganic compounds such as lithium compounds.

The formation of tar in carbonylation acetic anhydride processes and the problem of recovering catalyst value therefrom are described in U.S. Patent 4,388,217 and European Patent 255,389. U.S. Patent 4,388,217 further discloses a process for recovering catalyst values from such tar by submitting a catalyst-tar solution to an extraction using methyl iodide and aqueous hydrogen iodide. In the practice of the extraction process, a substantial amount of the rhodium present in the rhodium-tar solution is recovered in the aqueous hydrogen iodide phase which may be recycled to the carbonylation process. The presence of the hydrogen iodide in the aqueous phase stabilizes the water-soluble rhodium compound or compounds, thereby preventing the loss of insoluble rhodium which can plate out on the extraction equipment and/or the walls of pipes, vessels, etc. Most of the tar component of the catalyst-tar solution is recovered in the methyl iodide phase. After removal of the methyl iodide, for example, by distillation, the tar may be subjected to further treatment to recover any residual rhodium present therein and then disposed of by incineration. Procedures for recovering such residual rhodium are disclosed in U.S. Patents 4,364,907, 4,578,368, and 4,650,649.

The rhodium extraction efficiency achieved by means of the extraction process described in U.S. Patent 4,388,217 ranges from approximately 84% to 96% in laboratory batch experiments. Operation of the extraction process in a continuous or semi-continuous mode of operation using 51% aqueous hydrogen iodide as the aqueous extractant gave a rhodium extraction efficiency of approximately 85%.

EP-A-200 023 describes a non-aqueous extraction process wherein a catalyst-tar solution is subjected to extraction with a dialkyl ether to free the catalyst-tar solution from the tar, acetic acid, acetic anhydride, and ethylidene diacetate and separating the ether phase from a precipitated promoter-containing catalyst complex. The remaining ether phase then is treated with iodine or methyl iodide to precipitate additional promoter-containing catalyst complex which is separated by filtration. The separated portions of promoter-containing catalyst complex are then combined with the acetic acid, acetic anhydride, and ethylidene diacetate recovered, and the resulting catalyst solution is returned to the carbonylation process after removing residual dialkyl ether by distillation. The process of EP-A-200 023 requires extreme care in the handling of the precipitated catalyst to avoid losses of expensive rhodium and means for the recovery and recycle of the dialkyl ether extraction agent.

We have discovered that the presence of elemental iodine in the rhodium-containing extraction mixture, i.e., the mixture containing the catalyst-tar solution which is submitted to the extraction process, increases substantially the rhodium extraction efficiency of the process described in U.S. Patent 4,388,217. Typically, the amount of rhodium present in the catalyst tar solution which can be recovered in the aqueous phase and recycled to the carbonylation process is at least 95% and may exceed 99%. The use of elemental iodine in the extraction process also permits the use of a lower concentration of hydrogen iodide in the aqueous extractant and provides a means for replenishing iodine to the carbonylation production system from which small but significant amounts of iodine are lost, for example, in the product(s), by-products and tar.

The process provided by this invention therefore comprises the recovery of rhodium catalyst values from a catalyst-tar solution derived from a production system in which acetic anhydride is prepared by contacting a mixture of methyl acetate and methyl iodide with carbon monoxide in the presence of a rhodium catalyst wherein the catalyst tar solution is extracted with a combination of methyl iodide and aqueous hydrogen iodide in the presence of elemental iodine and recovering the rhodium catalyst values in the aqueous phase, wherein the amount of elemental iodine added to the rhodium-containing extraction mixture is at least 1 mole I₂ per mole of rhodium, i.e., [Rh]. The upper limit of the amount of iodine which may be used is limited only by practical considerations such as solubility but typically will not exceed about 100 moles of I₂ per mole of [Rh]. Preferably, elemental iodine is used in an amount which gives an I₂:Rh mole ratio in the range of about 3:1 to 20:1.

The process in which the tar is formed comprises the preparation of acetic anhydride by contacting in the liquid phase a mixture of methyl acetate and methyl iodide with carbon monoxide in the presence of a rhodium catalyst at elevated pressure and temperature wherein a feed mixture of methyl acetate and methyl iodide is continuously fed to a carbonylation reactor and a reaction mixture containing acetic anhydride is continuously removed. Optionally, the rhodium-catalyzed process may be carried out in the presence of one or more inorganic or organic promoters such as those described in the patents cited hereinabove. In the practice of the process, the feed to the reactor is such as to maintain within the reaction mixture about 250 to 1300 ppm, preferably about 500 to 1000 ppm, rhodium and about 7 to 35 weight percent methyl iodide. The remainder of the reactor contents consists primarily of methyl acetate, catalyst promoters and acetic anhydride product with minor amounts of by-products such as ethylidene diacetate and acetone. The reactor feed optionally may contain a solvent such as acetic acid in an amount which will maintain about 5 to 40 weight percent acetic acid in the reaction mixture. Up to about 7 volume percent of the carbon monoxide employed in the process may consist of hydrogen in accordance with the process disclosed in U.S. Patent 4,374,070. The above-described carbonylation processes include processes designed for the coproduction of acetic acid and acetic anhydride by the inclusion of water and/or methanol in the reactor feed, e.g., as described in European Patent 87,870, and for the production of ethylidene diacetate and the coproduction of ethylidene diacetate and acetic anhydride by contacting methyl acetate and methyl iodide with a mixture of hydrogen and carbon monoxide, e.g., as described in Belgian Patent 839,321.

The carbonylation process may be carried out in a liquid or vapor take-off mode of operation. In liquid take-off operation, the catalyst components, i.e., the rhodium and promoter(s), methyl iodide and unreacted methyl acetate along with acetic anhydride are recovered from the reactor effluent and recycled. When necessary, fresh rhodium, as rhodium chloride, rhodium acetate or other rhodium compound, and promoter(s) are added to the catalyst recycle. The fresh catalyst components may be conveniently added as a solution in acetic acid. In a vapor take-off system, all or essentially all of the catalyst components remain in the reactor and thus, the risk of their depletion is reduced considerably.

Normally, the tar formed during the carbonylation reaction is removed continuously or intermittently from the carbonylation system in the form of a solution in a mixture of the compounds present in the system. The catalyst-tar solution may be removed either from the reactor or, in the case of a system employing a liquid product take-off from the reactor, from some point in the normal catalyst recycle stream. The solution can be submitted to the aqueous/organic solvent extraction or can be concentrated by stripping off some of the liquids present. In production facilities in which the rhodium is recycled to the carbonylation reactor, the tar-containing recycle stream normally will have been concentrated to some extent in the product recovery section of the facilities.

The amounts of aqueous hydrogen iodide and methyl iodide that can be used to satisfactorily remove the rhodium catalyst values from the catalyst-tar solution may vary substantially depending on a number of factors such as the concentration of the tar in the liquids removed from the production system, the extent to which the catalyst-tar solution has been concentrated, the amount of rhodium present in the tar and the type of apparatus that is utilized in the partitioning-extraction of the catalyst-tar solution. For example, the volume ratio of aqueous hydrogen iodide solution to catalyst-tar solution may be in the range of about 1:10 to 10:1. However, the use of ratios approaching 1:10 may render separation of the aqueous/organic phases difficult whereas the use of ratios approaching 10:1 will increase substantially the energy or materials required to remove or scavenge, e.g., using acetic anhydride, water prior to the recycle of the recovered rhodium values to the process. Thus, the volume ratio of aqueous hydrogen iodide solution to catalyst-tar solution usually is in the range of about 4:1 to 1:4. While the concentration of hydrogen iodide in the aqueous hydrogen iodide solution may vary from about 1 to 57 weight percent, the use of the highly concentrated solutions favored in the examples of U.S. Patent 4,388,217 is not necessary to achieving rhodium recovery efficiencies in the range of 95 to 99+ percent according to the process of our invention. Preferably, the process is carried out using an aqueous extractant containing about 5 to 25 weight percent hydrogen iodide.

The amount of methyl iodide employed should be at least 2 parts, and may be as high as 100 parts, by weight per part of concentrated catalyst-tar solution. Methyl iodide to concentrated catalyst tar solution ratios of about 3 to 50 normally give good results.

In the practice of our novel process, a solution containing tar and catalyst values is removed intermittently or continuously from the carbonylation system and fed to a hold tank. The tar solution then can be fed continuously or semi-continuously to a still-decanter fitted with means for agitation. The composition of the tar solution will vary depending, for example, upon the point from which it is taken from the carbonylation and the materials used in the particular carbonylation system. Initially, low boiling components such as methyl iodide and methyl acetate along with some acetic acid and acetic anhydride are removed to give a concentrated solution of the tar and rhodium values in a mixture consisting primarily of acetic anhydride and acetic acid. Methyl iodide and aqueous hydrogen iodide containing elemental iodine are added to the concentrated catalyst-tar solution with agitation. In batch operations, best results are obtained if the methyl iodide is added first to the catalyst-tar solution which has been cooled to just below the boiling point of methyl iodide. After partitioning, agitation is stopped, the aqueous and organic layers are allowed to separate and the methyl iodide phase containing the tar is removed from the bottom of the still-decanter. The remaining aqueous phase comprising the hydrogen iodide and rhodium values in water and acetic acid may be vacuum distilled to remove water. The catalyst solution then can be combined with the liquids initially removed and recycled to the carbonylation reactor.

The methyl iodide and tar may be separated by distillation and the viscous tar-methyl iodide residue can be treated further to recover any iodine and rhodium values present. The water and methyl iodide recovered from the extraction process may be recycled for use in the next extraction.

The use of elemental iodine is especially advantageous when the process is conducted in a continuous or semi-continuous manner by feeding catalyst-tar solution, aqueous hydrogen iodide and methyl iodide simultaneously to a mix tank and feeding the mixture to about the midpoint of an extraction column. An aqueous stream containing most of the rhodium catalyst values is removed from the top of the column and the methyl iodide containing the tar is removed from the bottom. Normally, additional hydrogen iodide and methyl iodide are added to the lower and upper portions, respectively, of the extraction column. The amount of elemental iodine required to achieve an improved rhodium extraction efficiency, preferably a rhodium extraction efficiency of 95 to 99+ percent, may be introduced at any point in the system. For example, a solution of elemental iodine in acetic acid, aqueous hydrogen iodide or, especially, methyl iodide may be added to the mix tank, to the extraction column or to both. Alternatively, the elemental iodine may be generated from the hydrogen iodide by contacting the aqueous hydrogen iodide with an oxidant, such as oxygen, an oxygen-containing gas, a peroxide, etc., either before or after the addition of the aqueous hydrogen iodide to the extraction system.

In a preferred mode of operating the process provided by this invention continuously or semi-continuously, a concentrated solution of the catalyst components and tar in a mixture of acetic acid, acetic anhydride, methyl acetate and, optionally, ethylidene diacetate is fed to an agitated extractor feed tank equipped with means for agitation and heat removal. A stream of an extraction mixture taken from the middle portion of the extraction column (described hereinafter) is fed to the feed tank to which methyl iodide may also be added. The feed tank mixture (extraction mixture) is fed to the middle portion of an extraction column equipped with means for agitation, e.g., reciprocating plate agitation means. Methyl iodide is fed to the upper portion, preferably near the top, of the extraction column and aqueous hydrogen iodide is fed to the lower portion, preferably near the bottom, of the column. The aqueous phase containing most of the catalyst values, preferably at least 95 weight percent of the rhodium catalyst fed, is removed from the upper portion of the extraction column and the methyl iodide phase containing essentially all of the tar is removed from the bottom portion. The elemental iodine is provided to the system as a solution, e.g., in aqueous hydrogen iodide or methyl iodide, which may be added to the extractor feed tank, to the extractor column or to both.

The process of our invention is further illustrated by the following examples. The catalyst-tar solution used in the example is derived from the acetic anhydride production system described hereinabove and in U.S. Patent 4,374,070.

### EXAMPLE 1

A catalyst recycle stream containing approximately 820 ppm rhodium, 1900 ppm lithium, 5 weight percent methyl iodide, 17 weight percent methyl acetate, 2 weight percent acetone, 32 weight percent acetic acid, 43 weight percent acetic anhydride, 0.7 weight percent and from 5 to 8 weight percent tar is processed through a single pass evaporator at a rate of 1.8 gallons (8.82 L) per minute. The stripped material (concentrated tar-catalyst solution) is fed at a flow rate of 0.2 to 0.3 gallons (0.79-1.14 L) per minute to an agitated extractor feed tank to which is also added a stream of methyl iodide containing 5.5 weight percent elemental iodine at a flow rate of 0.4 to 0.5 gallons (1.52-1.90 L) per minute and a stream taken from the midpoint of the extractor column at a flow rate of 1.0 gallons (3.79 L) per minute. The extraction mixture containing approximately 8.2 weight percent I₂ is fed at the rate of 1.6 to 1.8 gallons (6.06-6.82 L) per minute to the middle of an extractor column equipped with reciprocating plate agitation means. The rhodium is extracted with an aqueous hydrogen iodide solution wherein the hydrogen iodide concentration varies from 5 to 15 weight percent by feeding the solution to the bottom of the extractor at the rate of 0.27 gallons (1.02 L) per minute. Methyl iodide is fed at the rate of 0.10 to 0.15 gallons (0.38-0.57 L) per minute to the top of the extractor to extract the tar. The extractor underflow consisting primarily of the tar dissolved in methyl iodide exits the extractor at the rate of 0.4 to 0.5 gallons (1.52-1.90 L) per minute. The rate of the extractor overflow containing the rhodium dissolved in the aqueous phase is 0.4 to 0.5 gallons (1.52-1.90 L) per minute.

Elemental iodine exits the extractor in concentrations of 1.41 weight percent in the underflow and 7.9 weight percent in the overflow. The rhodium concentration is 1765 ppm in the overflow and 18 ppm in the underflow giving a rhodium extraction efficiency of 98.9%.

The above-described procedure is repeated except that no elemental iodine is added to the extractor feed tank. The concentration of elemental iodine in the extractor feed tank, the extractor underflow and the extractor overflow is 0.15 weight percent or less. The rhodium concentration is 900 ppm in the overflow and 1100 ppm in the underflow giving a rhodium extraction efficiency of 48%.

### EXAMPLE 2

The extraction procedure described in Example 1 is carried out continuously over an extended period of time with all, or essentially all, of the elemental iodine being provided in the aqueous hydrogen iodide fed near the bottom of the extractor. During the operation of the process, the amount of elemental iodine fed and the concentration of the aqueous hydrogen iodide fed to the extractor are varied. The amount of elemental iodine in the aqueous phase exiting the extractor (extractor overflow) and the weight percent of hydrogen iodide in the aqueous hydrogen iodide fed to the extractor are measured each day. The rhodium extraction efficiency (%) is determined each day by measuring the amount of rhodium in the extractor overflow and underflow. These values for 21 days of operation are shown in Table I.

**TABLE I**

| Day | % I₂ in Extractor Overflow | % HI in Extractor Feed | Rh Extraction Efficiency |
|---|---|---|---|
| 1 | 0.5 | 60.5 | 88.5 |
| 2 | 0.6 | 55.0 | 90.0 |
| 3 | 0.7 | 55.5 | 92.7 |
| 4 | 0.7 | 61.5 | 89.7 |
| 5 | 1.2 | 58.5 | 93.6 |
| 6 | 3.7 | 61.0 | 96.7 |
| 7 | 3.5 | 56.5 | 96.9 |
| 8 | 4.5 | 59.0 | 96.3 |
| 9 | 4.1 | 59.5 | 94.5 |
| 10 | 4.4 | 59.0 | 95.1 |
| 11 | 5.1 | 54.0 | 97.5 |
| 12 | 3.8 | 55.5 | 96.8 |
| 13 | 7.3 | 58.0 | 96.6 |
| 14 | 5.5 | 55.5 | 97.9 |
| 15 | 4.7 | 48.5 | 97.2 |
| 16 | 6.2 | 50.5 | 97.4 |
| 17 | 3.4 | 46.5 | 97.2 |
| 18 | 3.1 | 40.5 | 97.8 |
| 19 | 5.6 | 30.5 | 98.1 |
| 20 | 1.5 | 27.5 | 95.7 |
| 21 | 2.3 | 27.0 | 97.6 |

## Claims

1. Process for the recovery of rhodium catalyst values from a catalyst-tar solution derived from a production system in which acetic anhydride is prepared by contacting a mixture of methyl acetate and methyl iodide with carbon monoxide in the presence of a rhodium catalyst wherein the catalyst-tar solution is extracted with a combination of methyl iodide and aqueous hydrogen iodide in the presence of elemental iodine and recovering the rhodium catalyst values in the aqueous phase, characterized by including in the rhodium-containing extraction mixture at least 1 mole I₂ per mole of rhodium in the rhodium-containing extraction mixture.

2. Process according to Claim 1 wherein the elemental iodine present gives an I₂:Rh mole ratio of about 3:1 to 20:1.

3. Process according to Claim 1 wherein the elemental iodine present gives an I₂:Rh mole ratio of about 3:1 to 20:1 and the concentration of hydrogen iodide in the aqueous hydrogen iodide is about 5 to 25 weight percent.

4. Process for the recovery of rhodium catalyst values from a catalyst-tar solution derived from a production system in which acetic anhydride is prepared by contacting a mixture of methyl acetate and methyl iodide with carbon monoxide in the presence of a rhodium catalyst which comprises the steps of:
(i) continuously feeding catalyst-tar solution, aqueous hydrogen iodide, and methyl iodide to a mix tank;
(ii) continuously feeding the extraction mixture of (i) to an extraction column;
(iii) continuously removing aqueous hydrogen iodide containing rhodium catalyst values from the upper portion of the extraction column; and
(iv) continuously removing methyl iodide containing tar from the lower portion off the extraction column:
characterized in that the process is carried out in the presence of elemental iodine in an amount which gives an I₂:RH mole ratio of at least 1.

5. Process according to Claim 4 wherein the elemental iodine present gives an I₂:RH mole ratio of about 3:1 to 20:1; the concentration of hydrogen iodide in the aqueous hydrogen iodide is about 5 to 15 weight percent; and the elemental iodine is added to the mix tank, to the extraction column, or both.

6. Process according to Claim 4 wherein the elemental iodine present gives an I₂:Rh mole ratio of about 3:1 to 20:1; the concentration of hydrogen iodide in the aqueous hydrogen iodide is about 5 to 25 weight percent; and the elemental iodine is generated from the hydrogen iodide by contacting the aqueous hydrogen iodide with an oxidant.

7. Process according to Claim 4 wherein the elemental iodine present gives an I₂:Rh mole ratio of about 3:1 to 20:1; the concentration of hydrogen iodide in the aqueous hydrogen iodide is about 5 to 25 weight percent; and the elemental iodine is generated from the hydrogen iodide by contacting the aqueous hydrogen iodide with oxygen or an oxygen-containing gas.

## Patentansprüche

1. Verfahren zur Rückgewinnung von Rhodium-Katalysatorwerten aus einer Katalysator-Teerlösung, abgeleitet aus einem Produktionssystem, in dem Essigsäureanhydrid hergestellt wird durch Kontaktierung einer Mischung aus Methylacetat und Methyliodid mit Kohlenstoffmonoxid in Gegenwart eines Rhodium-Katalysatoren, wobei die Katalysator-Teerlösung mit einer Kombination aus Methyliodid und wäßrigem Wasserstoffiodid in Gegenwart von elementarem Iod extrahiert wird und die Rhodium-Katalysatorwerte in der wäßrigen Phase wieder gewonnen werden, dadurch gekennzeichnet, da» in der Rhodium-enthaltenden Extraktionsmischung mindestens 1 Mol I₂ pro Mol Rhodium in der Rhodium-enthaltenden Extraktionsmischung eingeschlossen werden.

2. Verfahren nach Anspruch 1, wobei das vorhandene elementare Iod ein I₂:Rh-Mol-Verhältnis von etwa 3:1 bis 20:1 ergibt.

3. Verfahren nach Anspruch 1, wobei das vorhandene elementare Iod ein I₂:Rh-Mol-Verhältnis von etwa 3:1 bis 20:1 ergibt und die Wasserstoffiodidkonzentration in dem wäßrigen Wasserstoffiodid etwa 5 bis 25 Gew.-% ist.

4. Verfahren zur Rückgewinnung von Rhodium-Katalysatorwerten aus einer Katalysator-Teerlösung, abgeleitet aus einem Produktionssystem, in dem Essigsäureanhydrid hergestellt wird durch Kontaktierung einer Mischung von Methylacetat und Methyliodid mit Kohlenstoffmonoxid in Gegenwart eines Rhodium-Katalysatoren, welches die Schritte umfaßt:
(i) Kontinuierliche Zuführung von Katalysator-Teerlösung, wäßrigem Wasserstoffiodid und Methyliodid in einen Mischtank;
(ii) kontinuierliche Zufuhr der Extraktionsmischung aus (i) zu einer Extraktionssäule;
(iii) kontinuierliche Entfernung des wäßrigen Wasserstoffiodids, enthaltend die Rhodium-Katalysatorwerte aus dem oberen Teil der Extraktionssäule; und
(iv) kontinuierliche Entfernung von Methyliodid enthaltendem Teer aus dem unteren Teil der Extraktionssäule:
gekennzeichnet dadurch, daß das Verfahren in Gegenwart von elementarem Iod in einer Menge durchgeführt wird, die ein I₂:Rh-Mol-Verhältnis von mindestens 1 ergibt.

5. Verfahren nach Anspruch 4, wobei das vorhandene elementare Iod ein I₂-Rh-Mol-Verhältnis von etwa 3:1 bis 20:1 ergibt; die Konzentration des Wasserstoffiodids in dem wäßrigen Wasserstoffiodid ist etwa 5 bis 15 Gew.-%; und das elementare Iod wird dem Mischtank, der Extraktionssäule oder beiden, zugeführt.

6. Verfahren nach Anspruch 4, wobei das vorhandene elementare Iod ein I₂:Rh-Mol-Verhältnis von etwa 3:1 bis 20:1 ergibt; die Konzentration an Wasserstoffiodid in dem wäßrigen Wasserstoffiodid ist etwa 5 bis 25 Gew.-%; und das elementare Iod wird aus dem Wasserstoffiodid durch Kontaktierung des wäßrigen Wasserstoffiodids mit einem Oxidationsmittel erzeugt.

7. Verfahren nach Anspruch 4, wobei das vorhandene elementare Iod ein I₂:Rh-Mol-Verhältnis von etwa 3:1 bis 20:1 ergibt; die Konzentration des Wasserstoffiodids in dem wäßrigen Wasserstoffiodid ist etwa 5 bis 25 Gew.-%; und das elementare Iodid wird aus dem Wasserstoffiodid durch Kontaktierung des wäßrigen Wasserstoffiodids mit Sauerstoff oder einem Sauerstoff-enthaltenden Gas erzeugt.

## Revendications

1. Procédé pour la récupération de quantités de catalyseur à base de rhodium à partir d'une solution de catalyseur-goudron provenant d'un système de production dans lequel on prépare de l'anhydride acétique en mettant en contact un mélange d'acétate de méthyle et d'iodure de méthyle avec du mono-oxyde de carbone en présence d'un catalyseur à base de rhodium, dans lequel on extrait la solution de catalyseur-goudron avec une combinaison d'iodure de méthyle et d'iodure d'hydrogène aqueux en présence d'iode élémentaire et on récupère les quantités de catalyseur à base de rhodium dans la phase aqueuse, caractérisé par l'incorporation dans le mélange d'extraction contenant du rhodium d'au moins une mole de I₂ par mole de rhodium dans le mélange d'extraction contenant du rhodium.

2. Procédé selon la revendication 1, dans lequel l'iode élémentaire présent donne un rapport molaire I₂:Rh d'environ 3:1 à 20:1.

3. Procédé selon la revendication 1, dans lequel l'iode élémentaire présent donne un rapport molaire de I₂:Rh d'environ 3:1 à 20:1 et la concentration d'iodure d'hydrogène dans l'iodure d'hydrogène aqueux est d'environ 5 à 25% en poids.

4. Procédé pour la récupération de quantités de catalyseur de rhodium à partir d'une solution de catalyseur-goudron provenant d'un système de production dans lequel on prépare de l'anhydride acétique en mettant en contact un mélange d'acétate de méthyle et d'iodure de méthyle avec du mono-oxyde carbone en présence d'un catalyseur à base de rhodium qui comprend les étapes:
(i) à alimenter en continu une solution de catalyseur-goudron, de l'iodure d'hydrogène aqueux et de l'iodure de méthyle à un réservoir de mélange;
(ii) à alimenter en continu le mélange d'extraction de (i) à une colonne d'extraction;
(iii) à retirer en continu de l'iodure d'hydrogène aqueux contenant des quantités de catalyseur à base de rhodium de la partie supérieure de la colonne d'extraction; et
(iv) à retirer en continu de l'iodure de méthyle contenant du goudron à partir de la partie inférieure de la colonne d'extraction:
caractérisé en ce que le procédé est réalisé en présence d'iode élémentaire en une quantité qui donne un rapport molaire I₂:Rh d'au moins 1.

5. Procédé selon la revendication 4, dans lequel l'iode élémentaire présent donne un rapport molaire I₂:Rh d'environ 3:1 à 20:1; la concentration d'iodure d'hydrogène dans l'iodure d'hydrogène aqueux est d'environ 5 à 15% en poids; et l'iode élémentaire est ajouté au réservoir de mélange, à la colonne d'extraction ou aux deux.

6. Procédé selon la revendication 4, dans lequel l'iode élémentaire présent donne un rapport molaire I₂:Rh d'environ 3:1 à 20:1; la concentration d'iodure d'hydrogène dans l'iodure d'hydrogène aqueux est d'environ 5 à 25% en poids; et l'iode élémentaire est créé à partir de l'iodure d'hydrogène en mettant en contact l'iodure d'hydrogène aqueux avec un oxydant.

7. Procédé selon la revendication 4, dans lequel l'iode élémentaire donne un rapport molaire I₂:Rh d'environ 3:1 à 20:1; la concentration d'iodure d'hydrogène dans l'iodure d'hydrogène aqueux est d'environ 5 à 25% en poids; et l'iode élémentaire est créé à partir de l'iodure d'hydrogène en mettant en contact l'iodure d'hydrogène aqueux avec de l'oxygène ou un gaz contenant de l'oxygène.
